Europäisches Patentamt

**European Patent Office**

Office européen des brevets

(11) Publication number: **0 232 121**
A2

(12) **EUROPEAN PATENT APPLICATION**

(21) Application number: **87300739.7**

(22) Date of filing: **28.01.87**

(51) Int. Cl.⁴: **D 01 F 9/00**
D 01 F 11/00, A 61 L 15/00,
C 08 B 37/00

(30) Priority: **29.01.86 GB 8602115**

(43) Date of publication of application:
**12.08.87 Bulletin 87/33**

(84) Designated Contracting States: **DE FR GB IT SE**

(71) Applicant: **COURTAULDS PLC**
**18, Hanover Square**
**London W1A 2BB (GB)**

(72) Inventor: **Burrow, Thomas Richard**
**207 Albany Road**
**Coventry West Midlands (GB)**

**Laity, Peter Raffaele**
**213 Sadler Road**
**Keresley Coventry (GB)**

(74) Representative: **Hale, Stephen Geoffrey et al**
**J.Y. & G.W. Johnson Furnival House 14/18 High Holborn**
**London WC1V 6DE (GB)**

(54) **Absorbent fibres and their production.**

(57) An absorbent material comprises cross-linked polysaccharide fibres having covalent chemical cross-links between hydroxyl groups. The polysaccharide is water-soluble in its non-cross-linked state, for example dextran. The fibres are water-insoluble but are capable of absorbing at least twice their weight of water to form a swollen gel.

The fibres can be produced by dissolving a carboxylate ester of the polysaccharide in an organic solvent, preferably with a latent cross-linking agent for the polysaccharide, extruding the solution through a jet into an aqueous medium to form fibres and subjecting the fibres to conditions which cause hydrolysis of the ester groups in the polysaccharide and cross-linking of the hydroxyl groups so formed by the latent cross-linking agent.

EP 0 232 121 A2

Description

Absorbent fibres and their production

This invention relates to water-absorbent materials useful in medical dressings. A widely used dressing for wounds, particularly for ulcers, is a cross-linked dextran which is capable of absorbing aqueous fluids to form a swollen gel. The cross-linked dextran is used as a powder. Although it is effective as a wound dressing. the powder is difficult to apply to any wounds other than those most easily accessible and the dressing is difficult to change. The cross-linked dextran is described in British Patent 974,054 and US Patent 3,042,667.

The present invention therefore seeks in one embodiment to provide an improved absorbent material more readily usable as a wound dressing.

An absorbent material according to the invention comprises a cross-linked polysaccharide which is water-soluble in its non-cross-linked state, and is characterized in_ that the cross-linked polysaccharide contains covalent chemical cross-links between hydroxyl groups and is in the form of fibres which are water-insoluble but are capable of absorbing at least twice their weight of water to form a swollen gel.

Dextran is the most preferred water-soluble polysaccharide; alternatives include starch, particularly amylose or a starch containing a high proportion of amylose, gellan, curdlan, pullulan and glycogen. The water-soluble polysaccharide is preferably of high molecular weight, e.g. above $10^5$, more particularly above $10^6$; for example the dextran used is preferably native dextran of molecular weight above 1,000,000.

The cross-linked fibres may also contain a cross-linked polysaccharide which is water-insoluble in its non-cross-linked state, especially cellulose, for example in amounts up to 80 per cent by weight based on total polysaccharide, especially 20 to 80 per cent by weight when the water-soluble polysaccharide is dextran and 5 to 80 per cent by weight when other water-soluble polysaccharides are used. The cross-links in the cross-linked soluble and insoluble polysaccharides may be between hydroxyl groups in the same or other molecules of the same type or between hydroxyl groups of molecules of different types.

Direct spinning of a solution of a water-soluble polysaccharide such as a dextran generally does not produce continuous fibres. The cross-linking reaction described in British Patent 974,054 does not proceed quickly enough to serve as the coagulating step in a wet-spinning process. The cross-linked polysaccharides are generally insoluble in all inert solvents so that spinning after cross-linking is precluded.

The present invention therefore seeks in a further embodiment to provide a process for producing absorbent cross-linked polysaccharide fibres.

A process according to the invention for the production of absorbent cross-linked polysaccharide fibres comprises:

(a) dissolving a carboxylate ester of a polysaccharide which is water-soluble in its unesterified state in an organic solvent,

(b) extruding the resulting solution through a jet into an aqueous medium to form fibres, and,

(c) simultaneously with or subsequently to fibre formation, subjecting the fibres to conditions which cause hydrolysis of the ester groups in the poly saccharide carboxylate ester and cross-linking of hydroxyl groups so formed.

The cross-linking of the hydroxyl groups is achieved by means of a cross-linking agent which is preferably incorporated in the organic solvent in step (a) as a latent cross-linking agent for the polysaccharide.

The polysaccharide carboxylate ester is preferably formed by reacting a polysaccharide with a monocarboxylic acid or anhydride or acid chloride thereof to convert at least a portion of the hydroxyl groups of the polysaccharide to carboxylate ester groups. The polysaccharide carboxylate ester can for example be an acetate, propionate, butyrate or benzoate or may contain more than one type of carboxylate group. Acetate groups are preferred to higher aliphatic carboxylate groups not only as having better physical properties for fibre spinning but also on cost grounds. Aromatic carboxylate groups such as benzoate may be preferred because they are less readily hydrolysed or saponified, giving greater control of hydrolysis and cross-linking and less loss of polysaccharide in the spinning bath. The polysaccharide carboxylate ester preferably has a degree of substitution of at least 1.5 carboxylate groups per hexose unit, for example 1.5 to 3, preferably 1.9 to 2.3, such groups per hexose unit. In the case of dextran the carboxylate ester preferably contains both acetate and benzoate groups. The degree of substitution with benzoate groups is preferably 0.3 to 1.0 benzoate groups per hexose unit.

Acetate groups are preferably formed by reacting the polysaccharide with acetic anhydride. The reaction is preferably carried out in a polar organic solvent inert to the anhydride, for example acetone or N-methyl pyrrolidone. The polysaccharide is suspended or dissolved in the solvent, for example at a concentration of 10- 30 per cent by weight, and the anhydride is added slowly to control the exothermic reaction. A catalyst for the esterification is preferably used, most preferably a neutral catalyst such as lithium chloride catalyst. Reaction in N-methyl pyrrolidone solution with lithium chloride catalyst is particularly preferred. A basic catalyst, for example a tertiary amine catalyst such as pyridine, can alternatively be used. A strong acid catalyst, such as sulphuric acid, can be used but may cause some depolymerisation of the polysaccharide. The reaction is preferably carried out at elevated temperature, for example 50-120°C, especially 80-120°C. Alternatively the polysaccharide can be suspended in the anhydride, but the reaction is then more difficult to control. Acetic acid can alternatively be used for esterification, with the use of a higher temperature and longer reaction time.

Benzoate groups are preferably formed by reaction with benzoyl chloride using a polar organic solvent inert to benzoyl chloride, for example acetone or preferably N-methyl pyrrolidone. When both acetate and benzoate groups are to be introduced, the polysaccharide is preferably first reacted with acetic anhydride to introduce the acetate groups and the product is then reacted with benzoyl chloride to introduce the benzoate groups. The amount of acetic anhydride reacted with dextran may for example be sufficient to form 0.9 to 2.0 acetate groups per hexose unit.

The polysaccharide carboxylate ester is dissolved in an organic solvent to form a spinning dope. The organic solvent is preferably a substantially neutral solvent such as acetone, dimethyl sulphoxide or N-methyl pyrrolidone. The amount of solvent is preferably sufficient to give a concentration of the polysaccharide carboxylate ester in the final spinning dope of 5 to 40 per cent by weight.

The spinning dope may additionally contain an ester of a water-insoluble polysaccharide, for example a cellulosEester such as a cellulose acetate which may for example have a degree of substitution of 1.5 to 2.5 acetate groups per hexose unit. The cellulose ester acts as a spinning aid, improving the strength of the fibres formed and reducing fibre breakage during spinning. The cellulose ester can for example be used at up to 80 per cent by weight based on the total polysaccharide ester in the spinning dope. To form cross-linked dextran fibres which can readily be made into fabrics it may be preferred to use cellulose acetate at 20-80 per cent by weight of total polysaccharide ester. Other water-soluble polysaccharides may require less cellulose ester, for example 5 to 20 per cent.

A latent cross-linking agent for hydroxyl groups of the polysaccharide is preferably incorporated in the spinning dope. By a latent cross-linking agent we mean an agent which is capable of cross-linking the polysaccharide under appropriate conditions but does not cross-link the polysaccharide ester in the spinning dope. The cross-linking agent is generally a bifunctional organic compound, both functional groups being capable of reacting with hydroxyl groups of the polysaccharide. Compounds containing at least one epoxide group are preferred, for example epichlorhydrin, diepoxybutane, diepoxyoctane, diglycidyl ether or the diglycidyl ether of ethylene glycol, propyleneglycol or 1,4-butanediol. The preferred cross-linking agent is epichlorhydrin which does not cross-link a polysaccharide ester in a neutral or acidic spinning dope even though the polysaccharide contains some unesterified hydroxyl groups. The concentration of latent cross-linking agent in the spinning dope is preferably 15 to 100 per cent by weight based on the total polysaccharide ester, higher proportions within this range being preferred when the concentration of polysaccharide ester in the dope is low.

The spinning dope is extruded into an aqueous medium to precipitate the polysaccharide ester in fibrous form. The aqueous medium is preferably alkaline, for example it may be a solution of sodium hydroxide or potassium hydroxide. The alkali facilitates the formation of continuous fibres. It also hydrolyses the carboxylate ester groups in the polysaccharide ester so that the hydroxyl groups of the polysaccharide are regenerated. It also catalyses the cross-linking of the polysaccharide by a bifunctional compound containing at least one epoxide group such as epichlorhydrin. The cross-linking thus proceeds simultaneously with the regeneration of hydroxyl groups in the polysaccharide. The concentration of sodium or potassium hydroxide in the aqueous medium is preferably 0.4 - 2.5 M. The aqueous alkali is preferably at above ambient temperature to give more rapid cross-linking and strengthening of the fibres, for example at a temperature of 50 - 100°C, most preferably 75 - 95°C. The residence time of the fibres in contact with the aqueous alkali is preferably 2 to 15 minutes at such temperatures; longer residence times may be required if the bath temperature is lower. Longer residence times may also be required for hydrolysis and cross-linking when the polysaccharide carboxylate ester contains a substantial proportion of benzoate groups. Such filaments can for example be soaked in 0.4 - 2.5 M sodium or potassium hydroxide at ambient temperature for 0.5 to 2 hours after formation.

The covalent chemical cross-links formed may be between hydroxyl groups in the same polymer chain (intramolecular) or between hydroxyl groups in different polymer chains (inter-molecular). The applicants believe that the cross-linking is mainly intramolecular. In any case the cross-linking leads to a water-insoluble but water-swellable fibre. If a cellulose ester is present in the spinning dope it is precipitated in the fibre with the main polysaccharide. The cellulose ester groups are also hydrolysed and the hydroxyl groups formed react with the cross-linking agent to form cross-linked cellulose. The cellulose may become cross-linked with the water-soluble polysaccharide.

The spinning dope is preferably extruded through a multi-hole spinneret directly into the aqueous alkaline medium to form a multifilament yarn and tow. A preferred method of spinning uses a cocurrent spin bath in which a stream of aqueous alkali flowing in the direction of travel of the fibres surrounds them in the spinneret. The fibres and alkali flow together in a spin bath in the form of a duct.

The cross-linked polysaccharide fibres can be formed into a dressing by known techniques for forming non-woven fabrics, particularly wet-laid non-woven fabrics, and may have sufficient tensile properties to be formed into the dressing by weaving or knitting. The fibres are preferably capable of absorbing at least three times their weight of water. More linear polysaccharides such as amylose, gellan or pullulan may form cross-linked fibres which are stronger but less absorbent than those formed from dextran. The swollen fibre forms a gel which is porous to small molecules, for example water vapour. As well as their uses in medical dressings, the fibres have molecular sieve properties and may be useful in separating mixtures, for example biological liquids.

A preferred process for forming a nonwoven dressing is a wet-laying process in which a tow of the fibres is spread in a flow of liquid, for example in a fish tail device whose cross-section gradually broadens in one direction while narrowing in the direction at right angles to it, and fed onto a water-pervious support, for example a conveyor formed of wire or plastics mesh. The fibres are preferably overfed onto the support at an overfeed of at least 5 per cent, for example 10-50 per cent, so that the fibres cross over each other on the support. The fibres are then bonded at their points of contact where they cross over each other. If the liquid used in the wet-laying process is aqueous the fibres are in a swollen state and become bonded to each other on drying.

In a preferred procedure the cocurrent spin bath feeds the wet-laying process directly. The fibres may be fully cross-linked at the stage at which they are deposited on the pervious support or may be only partially cross-linked. In the latter case the fibrous mat on the support may be further treated with aqueous alkali, preferably at 50-100°C, before it is removed from the support.

In an alternative spinning procedure the solution of polysaccharide carboxylate ester can be extruded into a substantially neutral aqueous bath to form polysaccharide ester fibres. The fibres can be subsequently treated with aqueous alkali to hydrolyse the polysaccharide ester and to catalyse the cross-linking of the fibre. In this case the cross-linking agent, for example epichlorhydrin, may be present in the spinning dope or may be applied later with the alkali. Spinning into an aqueous alkaline bath is however preferred as the polysaccharide ester fibres formed by spinning into a neutral solution are brittle: moreover it is more convenient to achieve fibre formation, hydrolysis and cross-linking in a single alkaline bath.

The invention is illustrated by the following example:

Example

10 g dextran (native dextran of m.wt above 5,000,000), was dispersed in 100 ml N-methyl pyrrolidone, with 15 ml pyridine as a catalyst at 80°C. The dextran was acetylated by the addition of 13 ml of acetic anhydride, which was allowed to react over 1 hour. The dextran was then benzoylated by addition of 9 ml benzoyl chloride, which was allowed to react for 1 hour. The reaction mixture was then poured into 1 l water container 25 g $Na_2CO_3$ to remove unreacted reagents from the dextran ester. The ester was filtered off, washed with more water and then dried. The ester contained 1.5 acetate groups and 0.5 benzoate groups per glucose unit.

The dry ester was redissolved in N-methyl pyrrolidone to make a 10% by weight solution. 5% by weight epichlorhydrin and 10% by weight cellulose acetate, based on the solution, were also added to the mixture. The solution was spun through a plurality of 200 micron diameter orifices into 5% by weight aqueous NaOH at 70°C and the fibres were soaked for 80 mins in NaOH, then washed thoroughly with water.

The fibres were lightly swollen. When analysed by infra-red, the fibres showed a peak at about 860 $cm^{-1}$, which is ascribed to the alpha-$C_1$ configuration of the glucose residues of dextran.

The fibres were dried. They swelled reversibly in water to a gel form but remained as discrete fibres. The water absorbence was 400 per cent by weight based on the dry fibres.

Example 2

Repetition of Example 1 using amylose, gellan, curdlan, pullulan or glycogen of molecular weight above $10^5$ esterified with acetic anhydride and admixed with varying amounts of cellulose ester based on soluble polysaccharide ester gives comparable but not necessarily identical results.

**Claims**

1. An absorbent material comprising a cross-linked polysaccharide which is water-soluble in its non-cross-linked state, characterised in that the cross-linked polysaccharide contains covalent chemical cross-links between hydroxyl groups and is in the form of fibres which are water-insoluble but are capable of absorbing at least twice their weight of water to form a swollen gel.

2. An absorbent material according to claim 1, characterised in that the polysaccharide is dextran.

3. An absorbent material according to claim l, characterised in that the polysaccharide is amylose, gellan, curdlan, pullulan or glycogen.

4. An absorbent material according to any of claims 1 to 3, characterised in that the fibres also contain cellulose having covalent chemical cross-links between hydroxyl groups.

5. An absorbent material according to claim 4, comprising 20 to 80% by weight cross-linked dextran and 80 to 20% by weight cross-linked cellulose.

6. A process for the production of an absorbent material comprising a cross-linked polysaccharide, characterised by

(a) dissolving a carboxylate ester of a polysaccharide which is water-soluble in its unesterified state in an organic solvent,

(b) extruding the resulting solution through a jet into an aqueous medium to form fibres, and,

(c) simultaneously with or subsequently to fibre formation, subjecting the fibres to conditions which cause hydrolysis of the ester groups in the polysaccharide carboxylate ester and cross-linking of hydroxyl groups so formed.

7. A process according to claim 6, characterised in that the polysaccharide is dextran and the carboxylate ester has a degree of substitution of 1.9 to 2.3 carboxylate groups per hexose unit.

8. A process according to claim 7, characterised in that the carboxylate ester contains acetate groups and benzoate groups, the amount of benzoate groups being 0.3 to 1.0 benzoate group per hexose unit.

9. A process according to any of claims 6 to 8, characterised in that the organic solvent is N-methylpyrrolidone or acetone.

10. A process according to any of claims 6 to 9, characterised in that a latent cross-linking agent for hydroxyl groups of the polysaccharide is incorporated in the polysaccharide carboxylate ester solution.

11. A process according to claim 10, characterised in that the latent cross-linking agent is a bifunctional organic compound containing at least one epoxide group.

12. A process according to claim 11, characterised in that the latent cross-linking agent is epichlorhydrin.

13. A process according to any of claims 10 to 12, characterised in that the solution of the carboxylate ester and the latent cross-linking agent is extruded into aqueous alkali.

14. A process according to any of claims 6 to 13, characterised in that the solution of the soluble poly saccharide carboxylate ester also contains a dissolved cellulose ester.